# EUROPÄISCHE PATENTSCHRIFT

(11) **EP 2 338 436 B1**
(45) Veröffentlichungstag und Bekanntmachung des Hinweises auf die Patenterteilung: **11.02.2015**
(21) Anmeldenummer: 10015161.2
(22) Anmeldetag: 01.12.2010
(51) Int. Cl.: A61B 19/00

(54) **Haltevorrichtung für medizinische Instrumente**
Holder for medical instruments
Dispositif de retenue pour instruments médicaux

(30) Priorität: 23.12.2009 DE 102009060493
(43) Veröffentlichungstag der Anmeldung: 29.06.2011
(73) Patentinhaber: Karl Storz GmbH & Co. KG, 78532 Tuttlingen (DE)
(72) Erfinder: Stefan, Jochen, 78532 Tuttlingen (DE); Bacher, Uwe, 78532 Tuttlingen (DE)

(56) Entgegenhaltungen:
- WO-A2-01/78617
- US-A- 3 584 822
- US-A1- 2004 218 352
- US-A1- 2009 072 107

## Beschreibung

Die Erfindung betrifft eine Haltevorrichtung für medizinische Instrumente, mit einem Tragarm, an dem mindestens ein medizinisches Instrument festlegbar ist und mit mindestens einem Gelenk zur Positionierung des Tragarms und/oder des medizinischen Instruments, wobei das mindestens eine Gelenk als mit mindestens einer Lagerschale und einer Gelenkkugel versehenes Kugelgelenk ausgebildet ist, dessen Verdrehwinkel um die Längsachse des Gelenks durch eine Verdrehsicherung begrenzt ist.

Derartige Haltevorrichtungen sind bei der Durchführung chirurgischer Eingriffe häufig erforderlich, um medizinische Instrumente verschiedenster Art, wie beispielsweise Retraktoren, Kameras oder Endoskope, über einen längeren Zeitraum in einer bestimmten Position zu halten. Durch die gelenkige Ausgestaltung der Haltevorrichtungen ist es für den Chirurgen möglich, das von dem Tragarm gehaltene medizinische Instrument exakt zu positionieren und die eingestellte Position der Haltevorrichtung durch Arretieren des Gelenks bzw. der Gelenke des Tragarms zu fixieren.

Um die an der Haltevorrichtung festlegbaren medizinischen Instrumente und/oder Teile der Haltevorrichtung selbst, wie beispielsweise die Sperrvorrichtungen zum Fixieren und Lösen der Gelenke des Tragarms, mit Strom und/oder einem pneumatischen oder hydraulischen Druckmittel versorgen zu können, ist es aus der Praxis bekannt, Leitungen durch das Innere des Tragarms und auch durch die die Tragarmteile verstellbar miteinander verbindenden Gelenke zu führen.

Eine Haltevorrichtung mit durch die Kugelgelenke geführten Leitungen, ist beispielsweise aus der DE 295 21 305 U1 bekannt. Um die Leitungen davor zu schützen, dass dieses durch Drehung der Gelenke abgedreht oder anderweitig zerstört werden, weisen die Kugelgelenke dieser bekannten Haltevorrichtung eine Verdrehsicherung auf, über die der Verdrehwinkel der Gelenke um ihre Längsachse begrenzbar ist, also beispielsweise nicht über 360° verdrehbar ist. Diese bekannte Verdrehsicherung besteht aus einer in der Gelenkkugel ausgebildeten Umfangsnut sowie einem rechtwinklig zur Umfangsnut auf der Gelenkkugel angeordneten Verdrehsicherungsring, der über radial einwärts weisenden Stifte in der Umfangsnut gelagert ist. Hierdurch erzeugt eine Bewegung der Gelenkkugel über die in die Umfangsnut eingreifenden Stifte eine Drehbewegung des Verdrehsicherungsrings um die Längsachse des Gelenks. Zur Begrenzung des Verdrehwinkles ist am Verdrehsicherungsring als Anschlag ein radial nach außen abstehender Stift angeordnet, der bei der Verdrehung des Verdrehsicherungsrings gegen einen als Gegenanschlag ausgebildeten Sicherungsstift anläuft, der seinerseits fest in der Lagerschale des Kugelgelenks gelagert ist.

Mit dieser bekannten Konstruktion ist es zwar möglich, den Verdrehwinkel des Gelenks so zu begrenzen, dass die durch das Gelenk geführten Leitungen geschützt werden, jedoch weist diese bekannte Konstruktion das Problem auf, dass bei Diagonalbewegungen der Gelenkkugel, insbesondere bei zeitgleicher Drehbewegung um die Längsachse des Gelenks, das System klemmen kann oder durch Reibung der Stifte auf der Oberfläche der Gelenkkugel bzw. des Verdrehsicherungsrings in der Führungsbahn eine Schwergängigkeit des Gelenks bewirkt. Darüber hinaus weist diese bekannte Konstruktion den Nachteil auf, dass sie um die Gelenkkugel viel Bauraum benötigt.

Aus der US-A-2009/0072107 ist eine Vorrichtung zum exakten Positionieren von Instrumenten an einem MRI-Scanner bekannt. Die beschriebene Vorrichtung weist einen Tragarm auf, der aus mehreren Tragarmteilen aufgebaut ist, wobei die einzelnen Tragarmteile über Gelenke relativ zueinander verstellbar sind. Zur Ausbildung der Gelenke weisen die Tragarmteile an einem Ende eine gekrümmte Schulter auf und am anderen Ende ein teilkugelförmige Ausnehmung auf, in die die gekrümmte Schulter des nachfolgenden Tragarmteils in der Art einer Kugelgelenk-Lagerschale-Verbindung einsetzbar ist. Weiterhin sind in den Tragarmteilen Durchgangsbohrungen ausgebildet, die zur Aufnahme von Hülsen dienen, durch die Spannseile und andere Kabel geführt werden können. Aufgrund der parallel zueinander in einem jeden Tragarmteil angeordneten Hülsen lassen sich die Tragarmteile nur begrenzt um die Längsachse des Tragarms zueinander verdrehen. Diese Vorrichtung erweist sich als aufwändig und nicht als verdrehsicher genug.Davon ausgehend liegt der Erfindung die **Aufgabe** zugrunde, eine Haltevorrichtung für medizinische Instrumente der eingangs genannten Art zu schaffen, deren Verdrehsicherung ausreichend sicher ist und eine kompakte Bauweise des Kugelgelenks ermöglicht.

Die **Lösung** dieser Aufgabenstellung ist erfindungsgemäß durch die Merkmalsgesamtheit des Anspruch 1 gelöst. Dabei ist die Verdrehsicherung im Inneren der Gelenkkugel angeordnet ist.

Durch die erfindungsgemäße Verlagerung der Verdrehsicherung in das Innere der Gelenkkugel ist es möglich, ein besonders kompakt bauendes Kugelgelenk bereitzustellen, dessen Verdrehsicherung darüber hinaus vor Einflüssen von außen geschützt ist.

Weiterhin wird gemäß der Erfindung vorgeschlagen, dass die Verdrehsicherung aus einem fest mit der Gelenkkugel verbundenen Anschlag und einem fest mit dem Gelenkgehäuse verbundenen Gegenanschlag besteht. Sobald der an der Gelenkkugel angeordnete Anschlag gegen den gehäuseseitigen Gegenanschlag anläuft, wird die Verdrehbarkeit des Kugelgelenks gestoppt, um die durch das Gelenk geführten Leitungen vor einer Beschädigung zu schützen.

Zur Anordnung und Ausbildung des gelenkkugelseitigen Anschlags wird mit der Erfindung vorgeschlagen, dass der Anschlag in etwa auf Höhe des Zentrums der Gelenkkugel an der Innenseite der Ausnehmung angeordnet ist. Dabei ist der Anschlag als an einem in die Innenseite der Ausnehmung eingelassenen Ring angeordneter und radial nach innen weisender Vorsprung ausgebildet ist.

Der den Gegenpart zum gelenkkugelseitigen Anschlag bildende gelenkgehäuseseitige Gegenanschlag ist gemäß der Erfindung an einem fest mit dem Gelenkgehäuse verbundenen und in die Ausnehmung der Gelenkkugel hineinragenden Stab angeordnet.

Weiterhin wird mit einer bevorzugten Ausführungsform der Erfindung vorgeschlagen, dass zur Anordnung der Verdrehsicherung im Inneren der Gelenkkugel im von der Lagerschale umfangenen Teil der Gelenkkugel eine sich radial nach außen erweiternde trichterförmige Ausnehmung ausgebildet ist, wobei der Öffnungswinkel der trichterförmigen Ausnehmung vorzugsweise dem Öffnungswinkel der Lagerschale zur Umgebung entspricht, um die Verschwenkbarkeit des Kugelgelenks nicht zu beeinflussen.

Vorzugsweise sind der gehäusefeste Stab und der daran angeordnete Gegenanschlag so ausgebildet, dass das in der Gelenkkugel angeordnete freie Ende des Stabes kugelförmig ausgebildet ist und der Gegenanschlag als in Richtung der Längsachse des Stabes verlaufender und radial nach außen von dem kugelförmigen freien Ende des Stabes abstehender Steg ausgebildet ist.

Im montierten Zustand der Verdrehsicherung ist das kugelförmige freie Ende des Stabes vorteilhafterweise innerhalb des mit dem Anschlag versehenen Rings angeordnet ist, wobei der Innendurchmesser des Rings so bemessen ist, dass er, abgesehen von dem angeformten und radial nach innen weisenden Anschlag, größer ist als der Außendurchmesser des kugelförmigen Stabendes einschließlich des radial nach außen abstehenden Gegenanschlags.

Schließlich wird mit einer bevorzugten Ausführungsform der Erfindung vorgeschlagen, dass der Gegenanschlag als in eine Nut des Stabes einsetzbare Federscheibe ausgebildet ist.

Weitere Merkmale und Vorteile der Erfindung ergeben sich anhand der zugehörigen Zeichnungen, in denen ein Ausführungsbeispiel einer erfindungsgemäßen Haltevorrichtung für medizinische Instrumente nur beispielhaft dargestellt ist, ohne die Erfindung auf dieses Ausführungsbeispiel zu beschränken. In den Zeichnungen wird gezeigt:
- Fig. 1: eine Haltevorrichtung für medizinische Instrumente gemäß dem Stand der Technik;
- Fig. 2: eine Schnittdarstellung eines Kugelgelenks für eine erfindungsgemäße Haltevorrichtung für medizinische Instrumente und
- Fig. 3: eine schematische perspektivische Ansicht der Gelenkkugel des Kugelgelenks gemäß Fig.2.

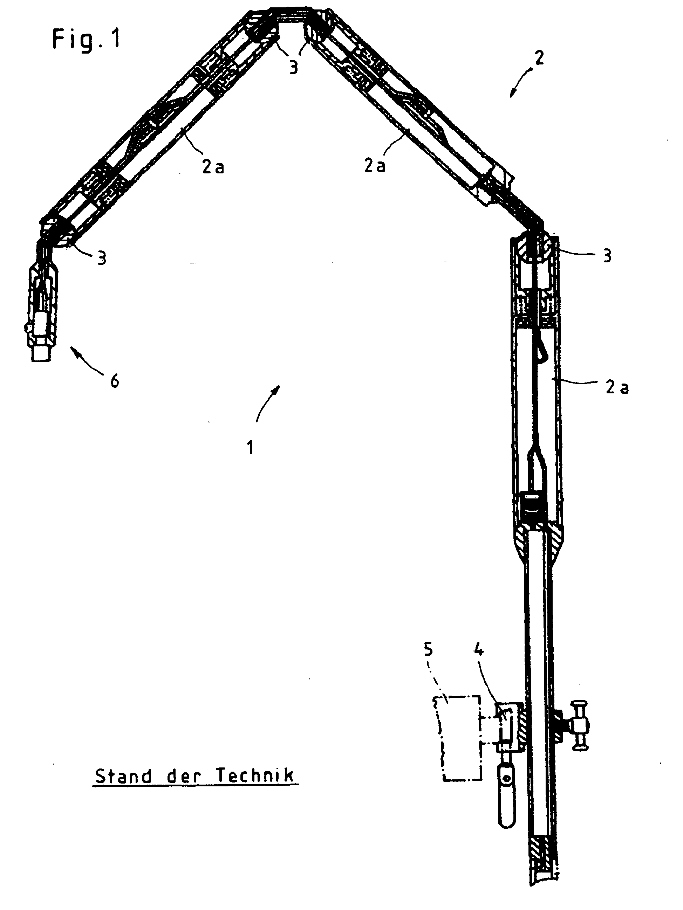

Die Abbildung Fig. 1 zeigt eine Haltevorrichtung 1 für medizinische Instrumente gemäß dem Stand der Technik.

Diese Haltevorrichtung 1 besteht im Wesentlichen aus einem aus mehreren Tragarmteilen 2a bestehenden Tragarm 2, wobei die einzelnen Tragarmteile 2a des Tragarms über als Kugelgelenke 3 ausgebildete Gelenke 3 relativ zueinander verschwenkbar miteinander verbunden sind.

Derartige Haltevorrichtungen 1 sind bei der Durchführung chirurgischer Eingriffe häufig erforderlich, um medizinische Instrumente verschiedenster Art, wie beispielsweise Retraktoren, Kameras oder Endoskope, über einen längeren Zeitraum in einer bestimmten Position zu halten. Durch die gelenkige Ausgestaltung der Haltevorrichtung 1 ist es für den Chirurgen möglich, das medizinische Instrument exakt zu positionieren und die eingestellte Position der Haltevorrichtung 1 durch Arretieren des Gelenks 3 bzw. der Gelenke 3 zu fixieren. Neben der endoskopischen Chirurgie finden derartige Haltevorrichtungen 1 auch in der offenen Chirurgie Anwendung.

Im Bereich seines proximalen Endes ist der Tragarm 2 über eine Einspannvorrichtung 4, beispielsweise am Operationstisch 5, festlegbar. Am distalen Ende weist der Tragarm 2 eine Instrumentenaufnahme 6 zur Aufnahme des über die Haltevorrichtung 1 zu positionierenden medizinischen Instruments auf.

Alternativ zu dem in Fig. 1 dargestellten Aufbau des Tragarms 2 aus mehreren hintereinander gekoppelten Tragarmteilen 2a, die über Gelenke 3 miteinander verbunden sind, ist es selbstverständlich auch möglich den Tragarm 2 mehrarmig so auszugestalten, dass ausgehend von einem Gelenk 3 mehrere Tragarmteile 2a in verschiedene Richtungen zeigen. Diese Tragarmteile 2a ihrerseits können wieder über Gelenke 3 mit weiteren Tragarmteilen 2a gekoppelt sein und an ihren distalen Enden jeweils mit Instrumentenaufnahmen 6 zur Aufnahme der über die Haltevorrichtung 1 zu positionierenden medizinischen Instrumente ausgestattet sein.

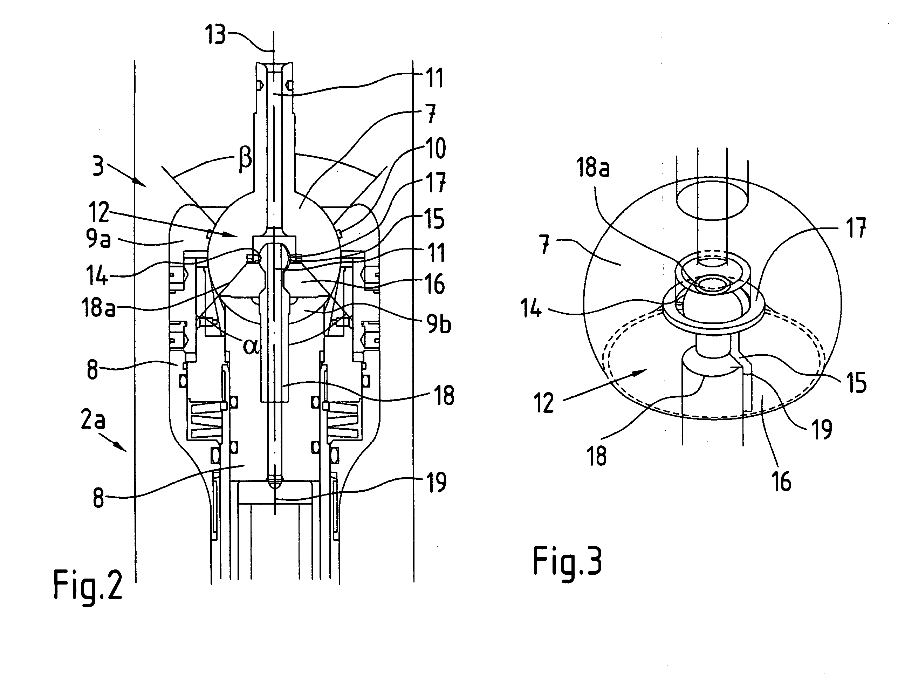

Der Aufbau der Kugelgelenke 3 ist den Abbildungen Fig. 2 und 3 zu entnehmen.

Wie aus Fig. 2 und 3 ersichtlich, bestehen die Kugelgelenke 3 aus einer Gelenkkugel 7, die verschwenkbar in einer in einem Gelenkgehäuse 8 ausgebildeten Lagerschale 9 gelagert ist. Bei dem dargestellten Ausführungsbeispiel ist die Lagerschale 9 aus einer oberen Lagerschale 9a und einer unteren Lagerschale 9b bestehend zweiteilig ausgebildet. Die Lagerschale 9 ist starr im Gelenkgehäuse 8 angeordnet, das heißt, dass die obere Lagerschale 9a und die untere Lagerschale 9b im montierten Zustand des Kugelgelenks 3 in Axialrichtung des Gelenks 3 nicht relativ zueinander bewegbar sind, also keine Vergrößerung oder Verringerung des Bewegungsspiels der Gelenkkugel 7 in der Lagerschale 9 aufgrund einer Bewegung der Lagerschale 9 möglich ist.

Wie weiterhin aus Fig. 2 und 3 ersichtlich, weist das Kugelgelenk 3 in der oberen Lagerschale 9a ein an der Gelenkkugel 7 anliegendes Dichtungselement 10 auf, um das Gelenk 3 nach außen und somit gegenüber äußeren Einflüssen, wie beispielsweise Feuchtigkeit und Schmutz, abzukapseln.

Um (nicht dargestellte) elektrische, pneumatische und/oder hydraulische Leitungen möglichst platzsparend entlang der Haltevorrichtung 1 zum in der Instrumentenaufnahme 7 gehaltenen medizinischen Instrument und/oder zu Arretiervorrichtungen der Gelenke 3 führen zu können, sind in den Gelenken 3 Führungskanäle 11 zur Aufnahme dieser Leitungen ausgebildet. Durch das Anordnen der Führungskanäle 11 und somit auch der Leitungen innerhalb der Gelenke 3 besteht aber die Gefahr, dass die Leitungen durch Drehung der Gelenke 3 abgedreht oder anderweitig beschädigt werden können. Zu diesem Zweck sind die Gelenke 3 mit einer Verdrehsicherung 12 ausgestattet, über die der Verdrehwinkel um die Längsachse 13 des Gelenks 3 begrenzbar ist, so dass das Gelenk 3 beispielsweise nicht über 360° verdrehbar ist.

Die Verdrehsicherung 12 des dargestellten Kugelgelenks 3 zeichnet sich dadurch aus, dass sie innerhalb der Gelenkkugel 9 des Kugelgelenks 3 angeordnet ist, wodurch eine besonders kompakte Bauart des Kugelgelenks 3 ermöglicht wird.

Bei der in Fig. 2 und 3 dargestellten Ausführungsform besteht die Verdrehsicherung 12 im Wesentlichen aus einem fest mit der Gelenkkugel 7 verbundenen Anschlag 14 und einem fest mit dem Gelenkgehäuse 8 verbundenen Gegenanschlag 15.

Zur Ausbildung der Verdrehsicherung 12 im Inneren der Gelenkkugel 9 ist im von der Lagerschale 9 umfangenen Teil der Gelenkkugel 7 eine sich radial nach außen erweiternde trichterförmige Ausnehmung 16 ausgebildet, wie sie insbesondere der Abbildung Fig. 2 zu entnehmen ist. Der Öffnungswinkel α der trichterförmigen Ausnehmung 16 entspricht dabei in etwa dem Öffnungswinkel β der Lagerschale 9 zur Umgebung, um ein einwandfreies Verschwenken der Gelenkkugel 7 in der Lagerschal 9 zu gewährleisten.

Der gelenkkugelseitige Anschlag 14 ist bei der dargestellten Ausführungsform an einem Ring 17 angeordnet, der in etwa in Höhe des Zentrums der Gelenkkugel 7 in die Innenseite der Ausnehmung 16 eingelassen ist. Der Anschlag 14 ist an dem Ring 17 als radial nach innen weisenden Vorsprung ausgebildet, der den Innendurchmesser des Rings 17 an der Stelle des Anschlags 14 um die Tiefe des Vorsprungs reduziert.

Der den Gegenpart zum Anschlag 14 bildende Gegenanschlag 15 ist bei der dargestellten Ausführungsform an einem fest mit dem Gelenkgehäuse 8 verbundenen Stab 18 angeordnet, der mit seinem freien Ende 18a in die trichterförmige Ausnehmung 16 der Gelenkkugel 7 hineinragt. Das freie Ende 18a des Stabs 18 ist dabei kugelförmig ausgebildet und so in der Mitte des mit dem Anschlag 14 versehenen Rings 17 angeordnet, dass der Ring 17 das kugelförmige freie Ende 18a des Stabs 18 konzentrisch umgibt. Der Gegenanschlag 15 ist als in Richtung der Längsachse 19 des Stabs 18 verlaufender und radial nach außen von dem kugelförmigen freien Ende 18a des Stabs 18 abstehender Steg ausgebildet.

Der den Gegenanschlag 15 bildende Steg kann beispielsweise als in eine Längs-nut 20 des Stabs 18 eingesetzte Federscheibe ausgebildet sein.

Die wie voranstehend beschrieben aufgebaute Verdrehsicherung 12 ist so innerhalb der Gelenkkugel 7 des Kugelgelenks 3 positioniert, dass einerseits aufgrund der trichterförmigen Ausgestaltung der Ausnehmung 16 das Schwenkverhalten des Kugelgelenks 3 in keiner Weise eingeschränkt wird, andererseits aber bei einer Drehung der Gelenkkugel 7 um die Längsachse 13 des Gelenks 3 diese Drehung durch die Verdrehsicherung 12 auf etwas unter 360° begrenzt wird.

Bei einer Drehung der Gelenkkugel 7 um die Längsachse 13 des Gelenks 3 dreht sich der fest mit der Gelenkkugel 7 verbundene Ring 17 mit der Gelenkkugel 7 frei um das kugelförmige freie Ende 18a des fest mit dem Gelenkgehäuse 8 verbundenen Stabs 18, bis der am Ring 17 angeformte Anschlag 14 gegen den am freien Ende 18a des Stabs 18 angeformten Gegenanschlag 15 anläuft. Durch diese Beschränkung des Verdrehwinkels der Gelenkkugel 7 um die Längsachse 13 des Gelenks 3 wird sichergestellt, dass innerhalb des Führungskanals 11 angeordnete Leitungen nicht überdreht und abgedreht oder anderweitig beschädigt werden können.

Eine wie voranstehend beschrieben ausgebildete Haltevorrichtung für medizinische Instrumente zeichnet sich dadurch aus, dass durch die Verlagerung der Verdrehsicherung 12 ins Innere der Gelenkkugel 7 das Kugelgelenk 3 insgesamt sehr kompakt gebaut werden kann. Darüber hinaus zeichnet sich die beschriebene Verdrehsicherung 12 dadurch aus, dass sie keine Bauteile aufweist, die permanent in Eingriff miteinander stehen, so dass es zu keiner Schwergängigkeit des Gelenks 3 aufgrund von gegenseitiger Reibung oder dergleichen kommen kann.

### Bezugszeichenliste

- 1: Haltevorrichtung
- 2: Tragarm
- 2a: Tragarmteil
- 3: Gelenk / Kugelgelenk
- 4: Einspannvorrichtung
- 5: Operationstisch
- 6: Instrumentenaufnahme
- 7: Gelenkkugel
- 8: Gelenkgehäuse
- 9: Lagerschale
- 9a: obere Lagerschale
- 9b: untere Lagerschale
- 10: Dichtungselement
- 11: Führungskanal
- 12: Verdrehsicherung
- 13: Längsachse
- 14: Anschlag

- 15: Gegenanschlag
- 16: Ausnehmung
- 17: Ring
- 18: Stab
- 18a: freies Ende
- 19: Längsachse
- 20: Längsnut

- α: Öffnungswinkel
- β: Öffnungswinkel

## Patentansprüche

1. Haltevorrichtung für medizinische Instrumente, mit einem Tragarm (2), an dem mindestens ein medizinisches Instrument festlegbar ist und mit mindestens einem Gelenk (3) zur Positionierung des Tragarms (2) und/oder des medizinischen Instruments, wobei das mindestens eine Gelenk (3) als mit mindestens einer Lagerschale (9) und einer Gelenkkugel (7) versehenes und in einem Gelenkgehäuse (8) angeordnetes Kugelgelenk (3) ausgebildet ist, dessen Verdrehwinkel um die Längsachse (13) des Gelenks (3) durch eine Verdrehsicherung (12) begrenzt ist, wobei die Verdrehsicherung (12) im Inneren der Gelenkkugel (7) angeordnet ist und aus einem fest mit der Gelenkkugel (7) verbundenen Anschlag (14) und einem fest mit dem Gelenkgehäuse (8) verbundenen Gegenanschlag (15) besteht,
**dadurch gekennzeichnet,**
**dass** der gelenkkugelseitige Anschlag (14) in etwa auf Höhe des Zentrums der Gelenkkugel (7) an der Innenseite einer Ausnehmung (16) der Gelenkkugel (7) angeordnet ist, wobei der Anschlag (14) als an einem in die Innenseite der Ausnehmung (16) eingelassenen Ring (17) angeordneter und radial nach innen weisender Vorsprung ausgebildet ist, und dass der gelenkgehäuseseitige Gegenanschlag (15) an einem fest mit dem Gelenkgehäuse (8) verbundenen, in die Ausnehmung (16) der Gelenkkugel (7) hineinragenden Stab (18) angeordnet ist.

2. Haltevorrichtung nach Anspruch 1, **dadurch gekennzeichnet, dass** im von der Lagerschale (9) umfangenen Teil der Gelenkkugel (7) eine sich radial nach außen erweiternde trichterförmige Ausnehmung (16) ausgebildet ist.

3. Haltevorrichtung nach Anspruch 2, **dadurch gekennzeichnet, dass** der Öffnungswinkel (α) der trichterförmigen Ausnehmung (16) dem Öffnungswinkel (β) der Lagerschale (9) zur Umgebung entspricht.

4. Haltevorrichtung nach Anspruch 1, **dadurch gekennzeichnet, dass** das in der Gelenkkugel (7) angeordnete freie Ende (18a) des Stabes (18) kugelförmig ausgebildet ist und der Gegenanschlag (15) als in Richtung der Längsachse (19) des Stabes (18) verlaufender und radial nach außen von dem kugelförmigen freien Ende (18a) des Stabes (18) abstehender Steg ausgebildet ist.

5. Haltevorrichtung nach Anspruch 4, **dadurch gekennzeichnet, dass** der Gegenanschlag (15) als in eine Längsnut (20) des Stabes (18) einsetzbare Federscheibe ausgebildet ist.

6. Haltevorrichtung nach Anspruch 1, **dadurch gekennzeichnet, dass** das kugelförmige freie Ende (18a) des Stabes (18) innerhalb des mit dem Anschlag (14) versehenen Rings (17) angeordnet ist.

## Claims

1. Holder for medical instruments, with a support arm (2) on which at least one medical instrument can be secured, and with at least one joint (3) for positioning the support arm (2) and/or the medical instrument, wherein the at least one joint (3) is designed as a ball-and-socket joint (3) which is provided with at least one bearing shell (9) and a joint ball (7) and is arranged in a joint housing (8), and whose pivot angle about the longitudinal axis (13) of the joint (3) is limited by a pivot guard (12), wherein the pivot guard (12) is arranged in the inside of the joint ball (7) and is composed of a stop (14) connected rigidly to the joint ball (7) and of a counter-stop (15) connected rigidly to the joint housing (8), **characterized in that** the stop (14) on the joint ball side is arranged on the inside of a recess (16) of the joint ball (7) approximately at the height of the centre of the joint ball (7), wherein the stop (14) is designed as a projection that points radially inwards and that is arranged on a ring (17) let into the inside of the recess (16), and **in that** the counter-stop (15) on the joint housing side is arranged on a rod (18) that is rigidly connected to the joint housing (8) and that protrudes into the recess (16) of the joint ball (7).

2. Holder according to Claim 1, **characterized in that** the part of the joint ball (7) surrounded by the bearing shell (9) has a funnel-shaped recess (16) that widens radially outwards.

3. Holder according to Claim 2, **characterized in that** the opening angle (α) of the funnel-shaped recess (16) corresponds to the opening angle (β) of the bearing shell (9) to the exterior.

4. Holder according to Claim 1, **characterized in that** the free end (18a) of the rod (18) arranged in the joint ball (7) has a spherical shape, and the counter-stop (15) is designed as a web that extends in the direction of the longitudinal axis (19) of the rod (18) and that juts out radially from the spherical free end (18a) of the rod (18).

5. Holder according to Claim 4, **characterized in that** the counter-stop (15) is designed as a spring washer that can be inserted into a longitudinal groove (20) of the rod (18).

6. Holder according to Claim 1, **characterized in that** the spherical free end (18a) of the rod (18) is arranged inside the ring (17) provided with the stop (14).

## Revendications

1. Dispositif de retenue pour instruments médicaux, comprenant un bras de support (2), sur lequel au moins un instrument médical peut être fixé et comprenant au moins une articulation (3) pour le positionnement du bras de support (2) et/ou de l'instrument médical, l'au moins une articulation (3) étant réalisée sous forme d'articulation à rotule (3) pourvue d'au moins une coque de palier (9) et d'une rotule d'articulation (7) et disposée dans un boîtier d'articulation (8), dont l'angle de rotation autour de l'axe longitudinal (13) de l'articulation (3) est limité par une fixation anti-rotation (12), la fixation anti-rotation (12) étant disposée à l'intérieur de la rotule d'articulation (7) et étant constituée d'une butée (14) reliée fixement à la rotule d'articulation (7) et d'une contre-butée (15) reliée fixement au boîtier d'articulation (8),
**caractérisé en ce que**
la butée (14) située du côté de la rotule d'articulation est disposée approximativement à hauteur du centre de la rotule d'articulation (7) sur le côté intérieur d'un évidement (16) de la rotule d'articulation (7), la butée (14) étant réalisée sous forme de saillie disposée sur une bague (17) encastrée dans le côté intérieur de l'évidement (16) et tournée radialement vers l'intérieur, et **en ce que** la contre-butée (15) située du côté du boîtier d'articulation est disposée sur une barre (18) reliée fixement au boîtier d'articulation (8) et pénétrant dans l'évidement (16) de la rotule d'articulation (7).

2. Dispositif de retenue selon la revendication 1, **caractérisé en ce qu'**un évidement (16) en forme d'entonnoir s'élargissant radialement vers l'extérieur est réalisé dans la partie de la rotule d'articulation (7) entourée par la coque de palier (9).

3. Dispositif de retenue selon la revendication 2, **caractérisé en ce que** l'angle d'ouverture (α) de l'évidement en forme d'entonnoir (16) correspond à l'angle d'ouverture (β) de la coque de palier (9) vers l'environnement.

4. Dispositif de retenue selon la revendication 1, **caractérisé en ce que** l'extrémité libre (18a) de la barre (18) disposée dans la rotule d'articulation (7) est réalisée sous forme sphérique et la contre-butée (15) est réalisée sous forme de nervure s'étendant dans la direction de l'axe longitudinal (19) de la barre (18) et faisant saillie radialement vers l'extérieur à partir de l'extrémité libre sphérique (18a) de la barre (18).

5. Dispositif de retenue selon la revendication 4, **caractérisé en ce que** la contre-butée (15) est réalisée sous forme de rondelle élastique pouvant être insérée dans une rainure longitudinale (20) de la barre (18).

6. Dispositif de retenue selon la revendication 1, **caractérisé en ce que** l'extrémité libre sphérique (18a) de la barre (18) est disposée à l'intérieur de la bague (17) pourvue de la butée (14).
